# NEUE EUROPÄISCHE PATENTSCHRIFT

(11) **EP 2 340 856 B2**
(45) Veröffentlichungstag und Bekanntmachung des Hinweises auf die Entscheidung über den Einspruch: **15.11.2017**
(45) Hinweis auf die Patenterteilung: 17.12.2014
(21) Anmeldenummer: 04078585.9
(22) Anmeldetag: 22.05.2004
(51) Int. Cl.: A61L 31/04, A61K 8/65, A61K 31/727, A61F 2/10, A61K 38/39, A61Q 5/00, A61L 27/00, A61K 45/06, A61Q 19/00, A61K 31/722, A61F 13/00, A61K 8/73, A61K 31/726

(54) **Kosmetische oder dermatologische Zubereitung umfassend eine Nährmedienphase**
Cosmetic or dermatologic formulation comprising a nutrient medium phase
Composition cosmétique ou dérmatologique comprennant une phase d'un milieu nutritif

(30) Priorität: 24.05.2003 DE 10323510; 24.11.2003 DE 10355110; 22.04.2004 DE 102004020035
(43) Veröffentlichungstag der Anmeldung: 06.07.2011
(73) Patentinhaber: La Prairie Group AG, 8604 Volketswil / Zürich (CH)
(72) Erfinder: Gohla, Sven, 21077 Hamburg (DE); Mönks, Monika, 33039 Nieheim (CH); Ibanez, Sybille, 8707 Uetikon am See (CH); Evangelisti, Carmen, 8427 Freienstein (CH)
(74) Vertreter: Hartmann, Jost

(56) Entgegenhaltungen:
- EP-A1- 0 296 078
- EP-A1- 0 462 426
- WO-A1-00/06608
- WO-A1-2004/103333
- DE-C1- 19 959 750
- US-A- 5 116 824
- US-A- 5 808 050
- US-A- 6 124 273
- US-B1- 6 432 710
- US-B1- 6 541 023
- Auszug aus der Mintel-Datenbank: Carita le Visage- Multi-Protection Facial Care, Oktober 2000 (Mintel-Eintragsnummer 75918)
- SKJÅK-BRAEK G. ET AL: 'Chitin and Chitosan', 1989, ELSEVIER APPLIED SCIENCE, LONDON pages 51 - 69
- Wikipedia-Auszug zum Stichwort "Hyaluronsäure"
- NutraWiki-Auszug zum Stichwort "Atelocollagen"
- Wikipedia-Auszug zum Stichwort "Kollagen"
- LI Q. ET AL: 'Applications and Properties of Chitosan', vol. CHPT. 1, 1996, GOOSEN M.F.A pages 3 - 29
- Wikipedia-Auszug zum Stichwort "Glykosaminoglykane"
- Auszug aus der Mintel-Datenbank: Juvena - Juvedical Cream, April 2004 (Mintel-Eintragsnummer 266975)
- Auszug aus der Mintel-Datenbank: Helena Rubinstein: Hydro-Urgenc Range Extension, Oktober 2000 (Mintel-Eintragsnummer 77388)
- Auszug aus der Mintel-Datenbank: Diadermine - Aqua Force Facial Gel Cream, August 2001 (Mintel-Eintragsnummer 109840)
- Schriftwechsel EPA, DPMA zu Prioritätsunterlagen
- Text der Prioritätsanmeldung DE 102004020035 - Kosmetische dermatologische Zubereitung umfassend eine Nährmedienphase

## Beschreibung

Die Erfindung umfasst eine kosmetische Zubereitung, umfassend Kollagenen, Chitosanen mit einem Acetylierungsgrad von bis zu 50% und Glykosylaminoglykan.

Innerhalb des Milieus des menschlichen Körpers existieren unterschiedliche Kreisläufe wie der Blutkreislauf, das Lymphsystem sowie die intra- bzw. extrazelluläre Gewebsflüssigkeiten. Die Zusammensetzung des Lösungsmittel "Wasser" mit seinen mineralischen und bio-organischen Bestandteilen in diesen unterschiedlichen "Transportmedien" sind annährend gleich und basieren, stark vereinfacht auf Salzen, Aminosäuren, Vitaminen, Zuckern, Proteinen und Proteiden, sowie Spurenelementen. Im Rahmen der Evolution hat unser Körper gelernt, innerhalb dieser Flüssigkeiten "Kommunikationsnetzwerke" und Ernährungsstrategien sowie ein Gleichgewicht von katabolen zu anabolen Vorgängen zu schaffen, welche das komplexe Leben unseres multizellulären Körpers erst ermöglichen. In dieser Umgebung hat unser Organismus gelernt, aus seinen "Einzelindividuen", den Zellen, ein kompliziertes, aber effizientes Netzwerk an direkten bzw. mediator-bedingten Kontakten zu knüpfen. Diese "Kommunikationswege" funktionieren nur dann effizient und unschadhaft, wenn das natürliche Fliessgleichgewicht unseres Körpers, die so genannte "Homöostase" besteht. Entfernt man Zellen aus dem Gewebsverband oder ist die Homöostase im Gewebsverband gestört, so können einzelne Zellen nicht mehr existieren bzw. Gewebe nicht mehr gesund funktionieren. Über Jahrzehnte haben die medizinischen und Biowissenschaften nach Möglichkeiten gesucht, um Gewebe bzw. einzelne Zellen in geeigneten Umgebungsbedingungen ausserhalb des Organismus zu kultivieren. Das gelang nur dadurch, das die Lebensbedingungen im Organismus für die zu kultivierenden Einzelzellen oder Gewebebestandteile möglichst perfekt nachgestellt werden konnten. Entfernt man also Zellen aus Gewebeverbund, so müssen sie in Umgebungen kultiviert werden, welche den natürlichen Lebensbedingungen im Körper möglichst nahe kommen. Dabei muss An- und Abtransport von Nährstoffen sowie die Präsenz von Vitalfaktoren gegeben sein.

Diese Umgebungen sind wohl definierte Zusammensetzungen aus mineralischen und Biostoffen, welche in der Wissenschaft als Zellkulturmedien bekannt sind. Zellkulturmedien sind im geeigneten Fachhandel als Pulver oder Flüssigmedien erhältlich und je nach Art der zu kultivierenden Zellen oder Gewebebestandteile leicht different zusammengesetzt. Zellkulturmedien werden in flüssiger Form angewendet, dabei ermöglicht es bei geeigneter Zusammensetzung den Erhalt oder gar die Vermehrung von Mikroorganismen oder Zellen in Kultur, also ausserhalb des Organismus. Im Zuge der Erkenntnisse in der Gewebeforschung konnten individuelle Bedürfnisse von Zellen bzw. Zellen in Gewebeverbunden herausgefunden und erforscht werden. Dabei ist das Verhältnis der mineralischen und bio-organischen Stoffe eines Zellkulturmediums von Zelltyp zu Zelltyp leicht variabel und für ein optimiertes Überleben und Wachstum exakt zu ermitteln. Grundsätzlich hängt die Zusammensetzung des Zellkulturmediums von den Bedürfnissen der zu vermehrenden Zellen ab. Man unterscheidet zwischen synthetischen Medien, deren Inhaltstoffe auf der Basis von Reinsubstanzen genau bekannt sind, und Komplexmedien, deren genaue Zusammensetzung schwanken kann und teilweise nicht genau bekannt ist. Zellkulturmedien enthalten neben Wasser zumeist eine Kohlenstoff- und eine Stickstoffquelle, Phosphat- und Schwefelverbindungen sowie Mineralstoffe und ggf. Wuchsstoffe oder Vitamine.

Sind die geeigneten Zusammensetzungen der Medien gegeben, so können sich die Zellen vermehren bzw. die für das Überleben notwendigen Faktoren "in situ" selber produzieren.

Um gutes Wachstum der Zellen zu generieren werden den Zellkulturmedien häufig Seren zugesetzt. Das Serum ist kompliziert zusammengesetzt und liefert den Zellen Hormone, Anheftungsfaktoren, Aminosäuren u.a. Kulturmedien die Seren enthalten sind jedoch teuer und erlauben keine thermische Sterilisierung. Man versucht daher mit Medien auszukommen, die keine Seren enthalten. Die serumfreien Kulturmedien ermöglichen die Kultivierung von Zellen unter kontrollierten und definierten Bedingungen, so dass unerwünschte Effekte durch Schwankungen der Serumzusammensetzung eliminiert werden. Zudem wird mit der Verwendung serumfreier Medien die Kontamination der Zellkulturen mit Viren und Bakterien vermindert.

Es ist bekannt, dass Hautzellen in ein-, zwei-, und dreidimensionalen Kulturen durch Optimierung der Bestandteile im Kulturmedium besonders schonend und lange am Leben erhalten bzw. sogar zur Vermehrung und Differenzierung gebracht werden können. Ferner konnte belegt werden, das geeigneten Medien auch die Produktion von Wachstumsfaktoren in situ ermöglichen.
Bei extremen Veränderungen der Haut, die durch großflächige Verbrennungen oder Verkühlungen entstehen, kann die Integrität sowie die Funktionalität des Hautgewebes so beeinträchtigt werden, das sie sich aus sich selber heraus nicht mehr regenerieren kann. Der Körper reagiert nach solch schweren Inzidenzen mit Hyperthermie, massiver Ausschüttung von Entzündungs- und Reizmediatoren sowie mit einem enormen Flüssigkeitsverlust, welcher in der Vergangenheit eigentlich immer unweigerlich zum Tod schwer verbrannter Personen geführt hat. Verbrennungen und Verkühlungen, welche zu Verlusten des Hautgewebes geführt haben, können dem Stand der Technik entsprechend durch Hauttransplantationen ausgeglichen und somit die Haut geschlossen werden. Das ist allerdings immer nur dann erfolgreich, wenn genug verbliebene Haut zur Transplantation vorhanden ist. In Fällen von Verbrennungen von mehr als 60% der Gesamthautgewebes kann in der Regel die Transplantation nicht allein helfen. Aus den verbliebenen Hautzellen muss taugliches Gewebe nachproduziert werden. Dabei kann aufgrund der Abstoßungsreaktionen zwischen nicht HLA-kompatiblen Geweben keine fremde Haut bzw. fremde Hautzellen genommen werden. Daher muss aus den verbliebenen lebensfähigen Hautzellen ein neues Hautgewebe in situ geformt wird.

Die verhornte Epidermis bildet den Schutzschild der Haut. Damit diese Funktion optimal erfüllt wird, müssen die Hautzellen (Keratinozyten) den Prozess der sogenannten epidermalen Differenzierung durchlaufen. Nach der Teilung der Zellen in der Basalschicht wandern die Keratinozyten zur Hautoberfläche und machen dabei eine Reihe von Veränderungen durch, bis sie als tote, flache, kernlose Korneozyten die Hornschicht (stratum corneum) bilden und schließlich abgeschuppt werden. Während der epidermalen Differenzierung werden verschiedenen Proteine mit spezifischen Funktionen ausgebildet. Hierzu zählen unter anderem Keratine, Involucrin, Filaggrin und T ransglutaminase. Zur optimalen Ausbildung der Epidermis und der Hornschicht ist es notwendig, dass diese Proteine koordiniert und in ausreichender Menge gebildet werden.

Aus dem Stand der Technik sind vielfach Kosmetika, Hautpflege- oder Wundheilpräparate bekannt, die Störungen der Haut ausgleichen oder zumindest vermindern helfen.
So wird beispielsweise die Altershaut kosmetisch in erster Linie mit Vitamin A-Derivaten oder Hydroxysäuren behandelt, die über eine Stimulation der Proliferation der Basalzellen in der Epidermis zu einer Verdickung der Epidermis und damit Glättung der Haut führen. Neuere Ansätze bestehen darin, die Proteine, die in der trockenen oder der Altershaut fehlen oder vermindert vorkommen, gezielt zu substituieren bzw. indirekt in die Stoffwechselprozesse einzugreifen, die bei trockener Haut oder mit zunehmendem Alter gestört sind, um diese zu normalisieren. Als Beispiel sei hier die Stimulation der Collagensynthese mit dem Zweck der Faltenreduzierung angeführt. Weiterhin werden beispielsweise Laminin, Substanzen zur Verlängerung der Lebenszeit von Hautzellen und bestimmte Extrakte zur Stimulierung der epidermalen Differenzierung eingesetzt. Hierbei handelt es sich teilweise jedoch um pharmakologisch wirksame Substanzen mit hohem Nebenwirkungspotenzial.

Die US 5,116, 824 richtet sich auf feste Wundauflagen enthaltend N-Succinylchitosan and Atelocollagen sowie optional Chondroitin-4-sulfonsäure.

Die US 5,166,187 befasst sich mit der Herstellung von biokompatiblen Protheseauflagen, die man mittels einer Lösung von Kollagen mit Chitosan und Glycosaminoglycan und anschließender Trocknung erreicht.

Keine der bekannten Zubereitungen aus dem Stand der Technik allein ermöglichen der Haut sich selber zu regenerieren ohne unerwünschte Nebenwirkungen aufzuweisen.

Die Aufgabe der vorliegenden Erfindung besteht darin eine Zubereitung zur Verfügung zu stellen, mit der es ermöglicht wird, dass die Haut sich selber regenerieren kann ohne unerwünschte Nebenwirkungen aufzuweisen.

EP 296078 beschreibt die Herstellung und Anwendung einer Mischung aus Kollagen, acetylierten Chitosan mit einem Acetylierungsgrad von 10 bis maximal 40% und Glykosylaminoglykan als künstliche Haut. Diese Mischung aus Biomaterialien findet Einsatz in der Orthopädie und in der plastischen Chirurgie sowie als Rekonstitutionsmatrizen zur Regeneration von Nerven-, Knochen- und Hautgewebe, letzteres insbesondere bei schwersten Verbrennungen.

Problem ist, das die in EP 296078 dargestellten Biomaterialien bislang nicht stabil in gängige kosmetische oder dermatologische Matrizes eingebaut werden konnten. Ebenso ist eine Darbietung als Kosmetikum oder als Wundpflegeprodukt bislang nicht beschrieben worden.

Gelöst werden die angeführten Aufgaben durch eine kosmetische oder dermatologische Zubereitung entsprechend dem Hauptanspruch. Gegenstand der Unteransprüche sind vorteilhafte Ausführungsformen der erfindungsgemäßen Zubereitung. Des weiteren umfasst die Erfindung die Verwendung derartiger Zubereitungen.

Es war überraschend und für den Fachmann nicht vorauszusehen, dass eine kosmetische und/oder dermatologische Zubereitung umfassend eine Mischung aus Kollagen, acetylierten Chitosan mit einem Acetylierungsgrad von maximal 50% und Glykosylaminoglykan auf Basis von wässrigen Gelen, Emulsionen von Olat, W/O/W- oder W/O-Typ, Mikroemulsionen oder kosmetischen Stiftpräparaten die gestellten Aufgaben löst. Insbesondere Zubereitungen enthaltend zusätzlich ein oder mehrere Hautzellkulturmedien, in denen das Medien bevorzugt gewählt wird aus DMEM/HAM's F-12 (1:1) und/oder MCDB 153, sind als besonders vorteilhaft zu bezeichnen. Darüber hinaus sind alle Kulturmedien als Zusatz denkbar, welche die Aufzucht gesunder differenzierter Haut ermöglichen (3-D-Modelle), also insbesondere Medien, die zur Aufzucht von primären Fibroblasten und/oder Keratinozyten dienen, ferner die vollständige Rekonstitution der Haut ermöglichen. Ferner sind Serumersatzstoffe für serumfreie Zellkulturen von Vorteil, aber nicht essentiell notwendig.
Bevorzugt ist der Zusatz zur erfindungsgemäßen Zubereitung an mindestens einer Verbindung, bevorzugt alle, gewählt aus der Gruppe L-Cystin, L-Glutamin, Hypoxanthin, L-Glutaminsäure, Thymidin, Glycin, Liponsäure, L-Histidin-HCl, Linolsäure, L-Isoleucin, Putrescin-2HCl, NaCl, Cholinchlorid, KCl, Putrescin, Natriumacetat, Vitamin B₁₂, Na₂HPO₄, Biotin, MgCl₂, Calciumpantothenat, CaCl₂, Nicotinamid, Glucose, Pyridoxin-HCl, Natriumpyruvat, Thiamin-HCl, NaHC03, Adenin, Phenolrot, myo-Inositol, HEPES, Liponsäure, Thymidin, L-Alanin, Folsäure, L-Arginin-HCl, Riboflavin, L-Asparagin, L-Asparaginsäure, CuSO₄, L-Cystein-HCl, FeSO4, L-Glutamin, MnSO4, L-Glutaminsäure, (NH₄)₆Mo₇O₂₄, Glycin, NiCl₂, L-Histidin-HCl, H₂SeO₃, L-Isoleucin, Na₂SiO₃, L-Leucin, SnCl₂, L-Lysin-HCl, NH₄VO₃, L-Methionin, ZnSO₄, L-Phenylalanin, L-Prolin, L-Serin, L-Threonin, L-Tryptophan, L-Tyrosin, L-Valin, FeSO₄, Insulin human, ZnSO₄, CoCl₂, CuSO₄, Na₂SeO₃, AlCl₃, CrK(SO₄)₂, NiCl₃, MnCl₂, EDTA.Na₂, Polysorbat 80, L-Leucin, Na₂HPO₄, L-Methionin, NaH₂PO₄, L-Phenylalanin, MgSO₄, MgCl₂, L-Serin, CaCl₂, L-Threonin, Fe(NO₃)₃, L-Tryptophan, FeSO₄, L-Tyrosin, NaHCO₃, α-Biotin, Na-Pyruvat, Folsäure, D-Ca-Pantothenat, L-Alanin, L-Arginin-HCl und/oder Pyridoxal-HCl.

Ein besonders vorteilhafte Kombination liegt in der Verknüpfung von zellernährenden Kulturmedien, vorzugsweise Medien zur Kultivierung von Haut- bzw. Corneakulturen aller Art mit einer zellulären Matrix enthaltend Kollagen, acetylierten Chitosan mit einem Acetylierungsgrad von bis zu 50%, bevorzugt bis 40%, und Chondroitinsulfaten. Diese Kombination allein, in Vermischung mit kosmetischen Zubereitungen oder Inkorporiert in Polyurethanmatrices erweist sich als äußerst effektiv bezüglich der Hautregeneration, Hautpflege und Wundheilung. Die Erfindung ermöglicht die Regeneration von Haut bzw. Teilhaut aus einzelnen Zellen (Dermis und Epidermis), sowie die Übertragung dieser in vitro vorkultivierten Gelmatrix auf geschädigtes Gewebe zur kompletten Hauterneuerung bzw. die Verhinderung bzw. Verminderung von Narbengewebe bei der Wundheilung. Die vorliegende Erfindung bietet ferner das ideale Umfeld (Matrix) zur Erneuerung der Haut bei topischer Applikation.

Die Herstellung der Matrix ist in EP 296078 beschrieben. Der Offenbarungsgehalt der EP 296078 ist hiermit im vollem Umfang Bestandteil der vorliegenden Erfindung.
Neu und für den Fachmann unvorhersehbar und somit erfindungsgemäss ist, das auch die Gewinnung der in der EP 296078 beschriebenen Matrix vollumfänglich durch maritime- und oder synthetische Rohstoffquellen erfolgen kann und zu gleichen Ergebnissen wie im Patent EP 296078 führt

Die erfindungsgemäße Zubereitung, auch als primär mikro- oder nanoporöse Matrix beschrieben, besteht bevorzugt aus maritimen Kollagenen, gewählt aus der Gruppe des Typ 3, Typ 1, Typ 4 und/oder Typ 5 oder Abmischungen derselben, Chitosanen, bevorzugt mit einem Molgewicht von 80.000D bis 1,5.000.000D, und einem Acetylierungsgrad von 5% bis zu 50%, in Abmischung mit einer Mischung aus Chondroitin 4- und -6- Sulfaten, welche bezogen auf die Ausgangsmenge der Kollagene zu 3 bis 15% eingesetzt werden. Die erfindungsgemässe Zubereitung oder Matrix kann man sich in Form eines mikro- bzw. nanotubulären Schwammes vorstellen.
Die Zusammensetzung der beschriebenen Moleküle erzeugt bei Gefriertrocknung einen Nano- bzw. Mikroschwamm (Matrix).

Die Matrix besteht aus einem durch Gefrietrocknung hergestellten Aerogel, welches in die Wasserphase(n), Wirkstoffphasen oder Medienphasen einer fertigen kosmetischen oder dermatologischen Zubereitungen eingetragen wird. Es wird dabei in ein Hydrogel umgewandelt bzw. als Aerogel in eine, oder zusammen mit einer, beispielsweise Polyurethan- oder Silikonmatrix, verarbeitet.
Es konnte gezeigt werden, das die erfindungsgemäße Kombination aus maritimen und/oder synthetischen Kollagenen, acetylierten Chitosanen und Glykosylaminoglykanen sowie insbesondere dem Zusatz an Zellkulturmedien Vorteile in Morphologie und Wachstumsrate von primären humanen Keratinozyten und Fibroblasten junger und alter Spendern in vitro ermöglicht. Dabei sind prinzipiell alle Wachstums- und Erhaltmedien möglich, vorzugsweise aber die, die auf die Bedürfnisse der Hautzellen abgestimmt sind und ferner den Aufbau "neuer Haut" aus einzelnen Dermis bzw. Epidermiszellen in der beschriebenen Matrix ermöglichen. Anwendungsstudien zeigen, das gereizte Haut sich bei der Behandlung mit der erfindungsgemäßen Matrix beruhigt. Dabei ist es insbesondere von Vorteil, dass die erfindungsgemässen Bestandteile Kollagen, Chitosan und Glykosylaminoglykan in einem ausgewogenen Verhältnis zueinader eingesetzt werden, insbesondere so wie es in EP 296078 beschrieben ist. Dies insbesondere, weil die Ausbildung eines mikro- bzw. nanotubulären Aerogels bzw. der Erhalt dieses Aufbaus in der kosmtischen Zubereitung oder Hautauflage nur durch die erfindungsgemäss aufgeführten spezifischen Wirkstoffe zueinander erfolgt.
Dabei wird die stationäre Biopolymerphase mit der oder den dispersen Phasen aus physiologischer Kochsalzlösung, Minimalmedien oder Vollmedien in eine Hydrogelphase umgewandelt Die Hydrogelphase mit den erfindungsgemässen Eigenschaften ergeben sich mit den erfindungsgemässen Matrixbausteine. Die einzelnen oder nur 2 der Wirkstoffe allein bzw. die Abmischung aller Wirkstoffe in nicht vorteilhaften Proportionen ergeben beispielsweise im Zusammenspiel mit den Zellkulturmedien als wässrige Phase bzw. im Zusammenspiel mit Polyurethan-Matrices nicht den gewünschten Effekt.

Erfindungsgemäß ist ein Verhältnis der Bestandteile Kollagen zu Chitosan von 60 bis 90% zu 40 bis 10%, insbesondere 75 - 85% zu 25 - 15%.

Kollagene ist eine Bezeichnung für eine Familie langfaseriger, linearkolloider, hochmolekularer Skleroproteine der extrazellulären Matrix, die in Bindegeweben (z. B. Haut, Knorpel, Sehnen, Bänder, Blutgefäße), in Ossein (der Protein-haltigen Grundsubstanz des Knochens) und im Dentin (Zahnbein) zusammen mit Proteoglykanen vorkommen. Sie gelten mit einem Anteil von 25-30% als die mengenmäßig häufigsten tierischen Proteine. Durch Fibronectin, das Kollagen u.a. Bestandteile der extrazellulären Matrix zu binden vermag, sich aber auch an Rezeptoren der Zelloberflächen anheftet, wird eine gegenseitige Verankerung der Kollagen-Fasern und der Zellen geschaffen. Die Zusammensetzung der Kollagene kann je nach Herkunft variieren. Man kennt Kollagene der Typen 1 bis 14, von denen jedoch nur die Typen I-III, V und XI die beschriebene Faserstruktur besitzen.

Die erfindungsgemäße Matrix ist Bestandteil von wässrigen Gelen, Emulsionen vom O/W-, W/O/W- oder W/O-Typ, Mikroemulsionen oder kosmetische Stiftpräparaten und kann somit erstmalig in gängigen kosmetischen Applikationsformen vertrieben werden.
Daneben ist auch die Bereitstellung in Hautauflagen, Patches, Pads, Tüchern oder Pflaster erfindungsgemäß. Hier insbesondere in Wundauflagen auf Basis von Polyurethanen.

Es kann sich dabei um eine professionelle Wundauflage handeln, die zur Therapie schwerster Brandverletzungen angewendet wird. Ebenso ist die home-in-use Anwendung möglich. In Form eines Aerogeles kann die Matrix auf die Wunde oder die zu behandelnde Hautpartie aufgelegt werden. Die Bestandteile der erfindungsgemäßen Matrix sind biologische Polymere, die durch ein spezifisches Mischungsverhältnis in ein stabiles Aerogel übertragen und sogar als stabiles Hydrogel rekonstituiert werden können. In zahlreichen Experimenten konnte gezeigt werden, das diese Gelmatrix komplette Heilhaut wieder aus einzelnen Hautzellen hervorbingt, wenn sie entsprechend auf die Wunde aufgebracht wird. Für die erfindungsgemäßen Formulierungen wurden Vorteile für Zellregeneration und Proliferation primärer Hautzellen vom Typ Keratinozyt bzw. Fibroblast festgestellt und experimentell belegt. Ferner konnten durch die Chondroitinsulfat, Chitosan- und Kollagenmatrix im Wechselspiel mit den Hautzellen, insbesondere in den 3-D-Hautmodellen, die Produktion von Elastin, Fibrillin und weiteren Biomarkern induziert werden, welche für die Qualität einer gesunden Haut verantwortlich sind. Ferner ist es möglich, durch das Zusammenspiel von Matrixmolekülen, wie oben beschrieben, und den Zellkulturmedien die retikuläre Verzapfung der Epidermis in der Dermis deutlich zu verbessern. Damit können durch das erfindunsgemäße Matrixmodell im Zusammenspiel mit den Kulturmedien eine ideale Regeneration der kompletten Haut aus nur wenigen Hautzellen erreicht werden bzw. eine bereits bestehende Haut mit der idealen, gesunden Wachstumsumgebung sowie Nährfaktoren versorgt werden. Im Zusammenspiel mit Polyurethan-Komponenten kann hier die Hautregeneration unter semi- bzw. okklusiven Bedingungen optimiert werden, was insbesondere Keloide und andere Narben zu normalisieren verhilft.

Der Prozess der Gewinnung der Bestandteile der beschriebenen Matrix besteht in dem Zusatz von acetyliertem Chitosan zu einer Kollagen/Wasser/ggf. Zellkulturmediumlösung und anschließendem Zusatz von Glykosylaminoglykan, insbesondere Chondroitin-4- oder -6-sulfat.

Die erfindungsgemäßen Proportionen der mindestens 3 Wirkstoffe der Matrix erlauben einen sustainded bzw. controlled release von Wirkstoffen, wie z.B. Q10, Retinol, AHA etc. und vermindert darüber hinaus ggf. die Nebenwirkungen dieser Agenzien.

Unter dem Begriff des Hautzellkulturmediums versteht der Fachmann alle flüssigen, pulverförmig oder festen Medien in oder auf dem sich einzelne Zellen vermehren können bzw. kultivieren lassen. Der Fachmann unterscheidet hierbei zwischen Reinigungsmedien, wie z.B. phosphat-gepufferte Kochsalzlösung, minimalen Erhaltmedien und sogenannten Vollmedien, in denen Zellen gesund und stoffwechserlaktiv sind. Vollmedien können einersits mit Wachstumsfaktoren aus tiersichen Seren bzw. sogenannten synthetischen Serenersatztstoffen versetzt sein, um das Wachstum spezifischer Zellen zu verbessern oder gar erst zu ermöglichen. Für jeden Zelltypus; insbesonder aber die Kultur primärer Zellen, gibt es Medien bzw. Medienabmischungen, die Wachstum, Differenzierung oder Metabolismus der spezifischen Zellen besonders gut unterstützen. Die erfindungsgässe Zusamenstellung aus Kollagenen, Chitosanen und Chondroitinsulfaten umfasst die Abmischung mit allen Reinigungs-, Minimal- bzw. Vollmedien, insebesondere aber Vollmedien, die zur Aufzucht von Hautzellen zusammengestellt wurden und im speziellen als Nährmedien für primäre humane Fibroblasten- bzw. Keratinozyten dienen und die als Nährmedien die Neubildung von Dermis aus einzelnen Fibroblasten bzw. Epidermis aus einzelnen Keratinozyten ermöglichen. Die erfindungsgemäßen Hautzellkulturmedien sind damit insbesondere zur Kultivierung von Hautzellen geeignet. Im speziellen sind die erfindungsgemäßen Hautzellkulturmedien beschrieben, welche in der Zusammensetzung ihrer Einzelbestandteile für folgende Zwecke geeignet sind:
- Kultur von Fibroblastenzellen
- Kultur von Keratinozytenzellen
- Co-Kulturen aus Keratinozyten und Fibroblasten
- Co-Kulturen aus Fibroblasten/Keratinozyten und weiteren hautrelevanten Zellen, wie Immunzellen , Melanozyten etc.
- Kulturmedien zur Generierung von dreidimensionalen Hautmodellen

Die erfindungsgemäßen Medien wirken auf Keratinozyten/Fibroblaste Mischkulturen und 3-D Hautmodellen. Überrachenderweise konnte gezeigt werden, dass kosmetische oder dermatologische Zubereitungen enthaltend die erfindungsgemäße Mischung aus Biomolekülen und Hautzellkulturmedien in oder auf der menschlichen Haut selber diejenigen Mechanismen zu aktivieren bzw. nachzustellen vermögen, welche die Haut für die Homöostase und gesunde Autopoiese gebrauchen. Dabei lassen sich Mischungen aus fibroblasten bzw. keratinozyten-relevanten Wachstumsmedien direkt oder in geeigneten Vesikeltechnologien einsetzen und für medizinisch/pharmazeutische Zwecke sowie kosmetische Zwecke verwenden.
Insbesondere haben sich sogenannte serumfreie Medien als vorteilhaft erwiesen, wenn die Zellfraktion der primären Keratinozyten und primären Fibroblasten im Sinne einer optimierten Homöostase positiv beeinflusst werden soll.

Der Einsatz der hautrelevanten Kulturmedien im Zusammenspiel mit den Matrixbiomolekülen bedingt die autologe, gesunde und individuelle Regeneration defizitärer Hautfunktionen in vitro, ex vivo und in vivo. So kann die Regeneration der Haut, die Hautstraffheit oder aber einfach nur der Beitrag zur Hautpflege signifikant verbessert werden.

Prinzipiell sind alle Hautzellkulturmedien für die Verwendung in kosmetischen Zubereitungen geeignet. Insbesondere sind diejenigen Hautzellkulturmedien geeignet, die in der Literatur zur Kultivierung von Haut- oder hautrelevanten Zellen, zur Behandlung von Hautirritationen und Verbrennungen eingesetzt werden. Insbesondere Medien zur Kultivierung von verbleibenden Zellen nach massiven Verbrennungen zeigen nach Auftrag der topischen Zubereitungen einen äußerst vorteilhaften Effekt.
Erfindungsgemäße vorteilhafte Hautzellkulturmedien sind Medien, die die Neogenese von Fibroblasten bzw. Keratinozyten allein oder in Mischkulturen erlauben bzw die Bildung und Passagierung von nicht gutartigen Zellen reduzieren..

Die Hautzellkulturmedien DMEM/HAM's F-12 (1:1) und MCDB 153 sind dabei für den erfindungsgemäßen Einsatz in kosmetischen Zubereitungen besonders geeignet.

Entsprechend dem Literaturhinweis aus Barnes D. and Sato G.; Anal. Biochem 102, 255 [1980] ist DMEM/HAM's F-12 (1:1) eine 1:1 Mischung, wobei durch Zugabe von Dulbecco's MEM (DMEM = Dulbesccos Modified Eagles medium) der Nährstoffgehalt des Ham's F-12 Mediums erhöht wird. Dieses Medium ist die Grundlage zur Kultivierung von Zelllinien für Humanproteine, wie beispielsweise Erythropoetin.
DMEM/HAM's F-12 (1:1) setzt sich zusammen aus (Angabe in mg/l):

| | | | |
|---|---|---|---|
| NaCl | 6999,5 | L-Leucin | 59 |
| KCl | 311,8 | L-Lysin-HCl | 91,25 |
| Na₂HPO₄ | 71 L-Methionin | 17,24 | |
| NaH₂PO₄ - H₂O | 62,5 | L-Phenylalanin | 35,5 |
| MgSO₄-7H₂O | 100 | L-Prolin | 17,25 |
| MgCl₂-6H₂O | 61 L-Serin | 26,25 | |
| CaCl₂ | 116,61 | L-Threonin | 53,5 |
| Fe(NO₃)₃-9H₂O | 0,05 | L-Tryptophan | 9 |
| FeSO₄-7H₂O | 0,417 | L-Tyrosin | 38,7 |
| CuSO₄-5H₂O | 0,00125 | L-Valin | 52,85 |
| ZnSO₄ - 7H₂O | 0,432 | | |
| D-Glucose | 3151 | Cholinchlorid | 9 |
| NaHCO₃ | 2438 | α-Biotin | 0,00365 |
| Na-Pyruvat | 55Folsäure | 2,65 | |
| Phenolrot | 12,5 | D-Ca-Pantothenat | 2,24 |
| | myo-Inositol | 12,6 | |
| L-Alanin | 4,5 | Nicotinamid | 2,02 |
| L-Arginin - HCL | 147,5 | Pyridoxcal - HCl | 2 |
| L-Asparagin - H₂O | 7,5 | Pyridoxin - HCL | 0,031 |
| L-Asparaginsäure | 6,65 | Riboflavin | 0,22 |
| L-Cystein - HCl | 15,75 | Thiamin - HCl | 2,17 |
| L-Cystin | 24Vitamin B₁₂ | 0,68 | |
| L-Glutamin | 365,3 | Hypoxanthin | 2,05 |
| L-Glutaminsäure | 7,35 | Thymidin | 0,37 |
| Glycin | 18,75 | Liponsäure | 0,11 |
| L-Histidin - HCl - H₂O | 31,5 | Linolsäure | 0,042 |
| L-Isoleucin | 54,5 | Putrescin - 2HCl | 0,081 |

MCDB 153 wird entsprechend Literaturhinweis Barnes D. and Sato G.; Anal. Biochem 102, 255 [1980] zur Kultivierung von humanen Keratinozyten eingesetzt. Ferner als Minimalmedium PBS, Phosphat-gepufferte Saline, mit pH Werten von 3,5 bis 8.
MCDB 153 setzt sich zusammen aus (mg/l):

| | | | |
|---|---|---|---|
| NaCl | 7599 | Cholinchlorid | 13,96 |
| KCl | 111,83 | Putrescin | 0,1611 |
| Natriumacetat - 3 H₂O | 500 | Vitamin 1312 | 4,07 |
| Na₂HPO₄ - 7H₂O | 536,2 | Biotin | 0,01 46 |
| MgCl₂ - 6H₂O | 122 | Calciumpantothenat | 0,258 |
| CaCl₂ - 2H₂O | 4,411 | Nicotinamid | 0,03663 |
| Glucose | 1081 | Pyridoxin - HCl | 0,06171 |
| Natriumpyruvat | 55Thiamin - HCl | | 0,3373 |
| NaHC03 | 1176 | Adenin | 24,32 |
| Phenolrot | 1,317 | myo-Inositol | 18,02 |
| HEPES | 6600 | Liponsäure | 0,2063 |
| Thymidin | 0,7266 | | |
| L-Alanin | 8,91 | Folsäure | 0,79 |
| L-Arginin - HCl | 210,7 | Riboflavin | 0,03764 |
| L-Asparagin | 15,01 | | |
| L-Asparaginsäure | 3,99 | CuSO₄-5H₂O | 0,0002496 |
| L-Cystein - HCl - H2O | 42,04 | FeSO4 - 7H2O | 1,39 |
| L-Glutamin | 877,2 | MnSO4 - 5H2O | 0,000241 |
| L-Glutaminsäure | 14,71 | (NH4)6Mo7O24 - 4H2O | 0,001236 |
| Glycin | 7,51 | NiCl2 - 6H2O | 0,0001188 |
| L-Histidin - HCl - H20 | 16,77 | H2SeO3 | 0,003869 |
| L-Isoleucin | 1,968 | Na2SiO3 - 9H2O | 0,1421 |
| L-Leucin | 65,6 | SnCl2 - 2H2O | 0,0001128 |
| L-Lysin - HCl | 18,27 | NH4V03 | 0,000585 |
| L-Methionin | 4,476 | ZnSO₄ - 7H₂O | 0,144 |
| L-Phenylalanin | 4,956 | | |
| L-Prolin | 34,53 | | |
| L-Serin | 63,06 | | |
| L-Threonin | 11,91 | | |
| L-Tryptophan | 3,06 | | |
| L-Tyrosin | 2,718 | | |
| L-Valin | 35,13 | | |

Der Vorteil der Medien DMEM/HAM's F-12 (1:1) und MCDB 153 ist, dass sie in kosmetischen oder dermatologischen Zubereitungen, die für die Kultivierung von Monolayer, zweidimensionalen und organotypischen Hautmodellen, besonders ausgewählt und geeignet sind und die in vitro und ex vivo Stimulation bzw. den Erhalt der hautspezifischen Biofunktionen erlauben.

Als Serumersatzstoffe können weiterhin Lösungen folgender Zusammensetzung A bzw. B vorteilhaft den Medien zugesetzt sein.

| Lösung A | Lösung B | | |
|---|---|---|---|
| Komponenten (1000x) | µM | Komponenten (1000x) | µM |
| FeSO₄-7H₂O | 3000 | Insulin human in 0,01 M HCl | 86 |
| ZnSO₄-7H₂O | 3000 | | |
| CoCl₂-6H₂O | 1000 | | |
| CuSO₄-5H₂O | 10 | | |
| Na₂SeO₃ | 10 | | |
| AlCl₃-6H₂O | 5 | | |
| CrK(S0₄)₂-12H₂0 | 1,4 | | |
| NiCl₃-6H₂O | 1 | | |
| MnCl₂-4H₂O | 1 | | |
| EDTA.Na₂ - 2H₂O | 30000 | | |
| Polysorbat 80 VG | 3820 | | |

Für die Herstellung der Flüssigmedien, wird gemäss Literaturangaben zumeist hochreines, pyrogenfreies Wasser eingesetzt, dieses entspricht der WFI - Qualität (water for injection) nach Pharmakopöe Europa. Die Flüssigmedien werden sterilfiltriert und abgefüllt, wobei die Anlagen und Herstellvorschriften, so ausgelegt sind, dass der Eintrag von Endotoxinen und Keimen weitgehend ausgeschlossen wird.

Die erfindungsgemäßen Medien weisen auch bei Änderungen der Medienzusammensetzungen, wie beispielsweise mit oder ohne Cholinchlorid, mit oder ohne H₂SeO₃, vorteilhafte Eigenschaften in Bezug auf die Hautregeneration auf.

Die Hautzellkulturmedien und die Mischung aus Biomolekülen (Kollagen/Chitosan/Glykosylaminoglykan) sowie ggf. Zusätze werden in kosmetischen Zubereitungen zu einem Anteil von bis zu 99,9 Gew.%, bezogen auf die Gesamtmasse der Zubereitung, eingemischt.

Unter kosmetischen Zubereitungen bzw. Matrices sind topische Zubereitungen zu verstehen, die dazu geeignet sind, die genannten Medien in feiner Verteilung und bevorzugt in einer durch die Haut resorbierbaren Form auf die Haut aufzubringen. Hierfür eignen sich z. B. wässrige und wässrig-alkoholische Lösungen, Sprays, Schäume, Schaumaerosole, Salben, wässrige Gele, Emulsionen vom O/W- oder W/O-Typ, Mikroemulsionen, hydrophile bzw. lipophile Patches oder kosmetische Stiftpräparate. Die Erfindung umfasst als Träger ein wässriges Gel, eine O/W-Emulsion, eine W/O/W-Emulsion oder eine Mikroemulsion. Im Sinne der Erfindung kann die Zubereitung auch in Körperreinigungsmitteln wie z. B. Seifen, Duschbädern, Shampoos u. ä. verwendet werden.

Insbesondere handelt es sich bei den kosmetischen Formulierungen um Hydrogele bzw. Emulsionen aller Art, vorzugsweise O/W Emulsionen.

Als Öl- oder Lipidphase können alle in der Kosmetik bekannten Lipide eingesetzt werden. Erfindungsgemäße als Emulsion vorliegende Zubereitung enthält einen oder mehrere Emulgatoren. Diese Emulgatoren können vorteilhaft gewählt werden aus der Gruppe der nichtionischen, anionischen, kationischen oder amphoteren Emulgatoren.

Neben Wasser und physiologisch geeigneten Lösungsmitteln können u.a. pflegende Bestandteile, Öle, Wachse, Fette, rückfettende Substanzen, Verdickungsmittel, Antioxidantien, Emulgatoren, als Sonnenschutzfilter geeignete Substanzen, Enzyme, Aminosäuren, Proteine, Polysaccharie und/oder Duftstoffe enthalten sein. Die Zubereitungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Geeignete Zubereitungen sind auch diejenigen, die für die professionelle Wundversorgung bzw. Wundheilung bzw. Reduktion von Operationsnarben o. ä. eingesetzt werden können, wie z.B Polyurethan-Zubereitungen in Kombination mit Chitosan/Collagen/Chondroitin-6-sulfat Schwämmen oder Lösungen.

Vorteilhaft erweisen sich als Zusätze spezielle Wirkstoffe, wie z.B. von Antioxidantien.
Vorteilhaft werden die zugesetzten Antioxidantien gewählt aus der Gruppe bestehend aus Aminosäuren (z.B. Glycin, Lysin, Arginin, Cystein, Histidin, Tyrosin, Tryptophan) und deren Derivate (als Salz-, Ester-, Ether-, Zucker-, Nukleotid-, Nukleosid-, Peptid- und Lipid-Verbidung), Imidazole (z.B. Urocaninsäure) und deren Derivate (als Salz-, Ester-, Ether-, Zucker-, Nukleotid-, Nukleosid-, Peptid- und/oder Lipid-Verbidung), Peptide wie D,L-Carnosin, D-Carnosin, L-Camosin, Anserin und deren Derivate (z.B. als Salz-, Ester-, Ether-, Zucker-, Thiol-, Nukleotid-, Nukleosid-, Peptid- und Lipid-Verbidung), Carotinoide, Carotine (z.B. α-Carotin, β-Carotin, ψ-Lycopin, Phytoen, ) und deren Derivate (z. B. als Salz-, Ester-, Ether-, Zucker-, Nukleotid-, Nukleosid-, Peptid- und/oder Lipid-Verbidung), Chlorogensäure und deren Derivate (als Salz-, Ester-, Ether-, Zucker-, Thiol-, Nukleotid-, Nukleosid-, Peptid- und/oder Lipid-Verbidung), Aurothioglucose, Propylthiouracil und andere Thiole (z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (als Salz-, Ester-, Ether-, Zucker-, Thiol-, Nukleotid-, Nukleosid-, Peptid- und/oder Lipid-Verbidung) sowie Sulfoximinverbindungen (z.B. Homocysteinsulfoximin, Buthioninsulfone, Penta-, Hexa-, Heptathioninsulfoximin) in sehr geringen verträglichen Dosierungen (z.B. pmol bis µmol/kg). Ferner (Metall)-Chelatoren (z.B. A poferritin, D esferral, L actoferrin, α -Hydroxyfettsäuren, Palmitinsäure, Phytinsäure) und deren Derivate (als Salz-, Ester-, Ether-, Zucker-, Thiol-, Nukleotid-, Nukleosid-, Peptid- und/oder Lipid-Verbidung), α -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon, Ubichinol, Plastochinon und deren Derivate (als Salz-, Ester-, Ether-, Zucker-, Thiol-, Nukleotid-, Nukleosid-, Peptid- und Lipid-Verbidung), Vitamin C und Derivate (z.B. Ascorbylpalmitat, Mg-Ascorbylphosphat, Ascorbylacetat), Tocopherole und Derivate (z.B. Vitamin-E-acetat), sowie Phenolische Verbindungen und Pflanzenextrakte, diese enthaltend, wie z. B. Flavonoide (z. B. Glycosylrutin, Ferulasäure, Kaffeesäure), Furfurylidenglucitol, Butylhydroxytoluol, Butylhydroxyanisol, Nordihydroguajakharzsäure, Nordihydroguajaretsäure, Trihydroxybutyrophenon und deren Derivate (als Salz-, Ester-, Ether-, Zucker-, Nukleotid-, Nukleosid-, Peptid- und Lipid-Verbidung). Harnsäure und deren Derivate, Mannose und deren Derivate (als Salz-, Ester-, Ether-, Zucker-, Thiol-, Nukleotid-, Nukleosid-, Peptid- und Lipid-Verbidung). Zink und dessen Derivate (z.B. ZnO, ZnSO₄), Selen und dessen Derivate (z.B. Selenmethionin, Ebselen), Stilbene und deren Derivate (z.B. Stilbenoxid, Trans-Stilbenoxid) und die erfindungsgemäß geeigneten Derivate (als Salz-, Ester-, Ether-, Zucker-, Thiol-, Nukleotid-, Nukleosid-, Peptid- und/oder Lipid-Verbidung) dieser genannten Wirkstoffe.

Ein zusätzlicher Einsatz eines Puffers kann nötig werden, um die Bestandteile der dispersen Phase zu stabilisieren. Bevorzugt werden hier phosphat-gepufferte Kochsalzlösungen oder Citratpuffer eingesetzt.

Neben den Antioxidantien oder aus der Gruppe derselben werden Kombination der erfindunsgemäßen Zubereitungen mit speziellen Inhaltstsoffe, die bevorzugt gewählt werden aus der Gruppe Q10, AGR, Zn-Orotrat, Carnitin, Kreatin und/oder Taurin favorisiert.

Als Anwendungsbereiche der erfindunsgemäßen Zubereitung haben sich besonders vorteilhaft die Pflege aller Hauttypen mit Ausnahme aller septischen Entzündungen bewährt, ferner Spezialanwendungen wie die Microdermaabrasion, Säurepeeling und Retinolbehandlungen. Hier ist die Hautregeneration und Beruhigung der erfindungsgemässen Zubereitungen spürbar.

Bevorzugt ist darüber hinaus die kosmetische Pflege der Haut, insbesondere zur Verbesserung der Schönheit.

Die Verpackung kann alle kosmetisch gängigen Darreichungssysteme umfassen, wie z.B. Tiegel, Pumpflaschen, Pipettenflaschen, Kartuschen oder Kapseln.

Bei problematischen Formulierungen, in denen das Zellmedium nicht eingearbeitet werden kann, gäbe es die Möglichkeit das Zellkulturmedium und kosmetische Produkt erst vor der Anwendung zu vermischen, durch spezielle Verpackungselemente, wie z.B. Doppelkartuschen mit Mischkopf, wie sie beispielsweise aus 2-Komponentenkleber bekannt sind. Die Verpackung des Zellkulturmediums könnte auch zum Nachfüllen konzipiert werden, damit nur frisches Produkt zur Anwendung kommt.

Vorteilhaft ist die Einbindung der erfindungsgemäßen Matrix in Polyurethanmatrices und deren Ausbildung als kosmetische Hautauflage, Wundauflage in Pflaster oder Bandagen oder als Pad. Zu den möglichen Polyurethanmatrizes sind insbesondere diejenigen aus den Schriften DE 42 33 289, DE 43 08 347, DE 43 08 445, DE 43 28 190 und DE 101 28 685 zu nennen.

Es folgen vorteilhafte Ausführungsbeispiele der vorliegenden Erfindung. Die Mengenangaben beziehen sich stets auf Gewicht%, sofern nichts Gegenteiliges angegeben ist.
In allen Zubereitungen kann das oben beschriebene Verhältnis der Matrixmoleküle Kollagen, Chitosan und Glykosylaminoglykan von 0,00001 Gew.% bis 99 Gew.% der Endformulierung betragen, vorzugsweise 0,0005 Gew.% bis 50 Gew.% und idealerweise 0,0015 bis 30 Gew.%, bezogen auf die Gesamtmasse der Zubereitung. Das Diserpsionsmittel "Kulturmedium" sollte einem osmotischen Druck einer 0,5 bis 2% Kochsalzlösung entsprechen, idealerweise aber dem physioloischen osmotischen Druck der menschlichen Gewebe, insbesondere der Haut entsprechen.

### Beispiele

### Beispiel 1

| Formula (INCI/CTFA adopted names) | % w/w |
|---|---|
| WATER (AQUA) | 66,9268 |
| TRIISOSTEARIN | 4,0000 |
| BUTYLENE GLYCOL | 3,0209 |
| PETROLATUM | 2,7000 |
| GLYCERIN | 2,5800 |
| CETEARYL ETHYLHEXANOATE | 2,2500 |
| HYDROGENATED COCO-GLYCERIDES | 2,0000 |
| CAPRYLIC/CAPRIC TRIGLYCERIDE | 2,0000 |
| CETEARYL ALCOHOL | 1,6000 |
| OLEA EUROPAEA (OLIVE) OIL UNSAPONIFIABLES | 1,5000 |
| PEG-30 STEARATE | 1,1000 |
| TOCOPHERYL ACETATE | 1,0000 |
| STEARIC ACID | 1,0000 |
| SORBETH-30 | 1,0000 |
| PALMITIC ACID | 1,0000 |
| CYCLOMETHICONE | 1,0000 |
| PHENOXYETHANOL | 0,7556 |
| STEARYL ALCOHOL | 0,5880 |
| POLOXAMER 188 | 0,5000 |
| BEESWAX (CERA ALBA) | 0,5000 |
| CETEARETH-20 | 0,4000 |
| SODIUM CARBOMER | 0,3300 |
| LANOLIN ALCOHOL | 0,3000 |
| CETYL ALCOHOL | 0,2640 |
| ISOPROPYL MYRISTATE | 0,2500 |
| GLYCERYL POLYMETHACRYLATE | 0,2150 |
| CETEARETH-25 | 0,2040 |
| METHYLPARABEN | 0,1526 |
| LECITHIN | 0,1490 |
| HISTIDINE | 0,1002 |
| SODIUM CITRATE | 0,1000 |
| DISODIUM EDTA | 0,1000 |
| CETEARETH-15 | 0,0900 |
| GLUCOSYLRUTIN | 0,0850 |
| BUTYLPARABEN | 0,0417 |
| ETHYLPARABEN | 0,0402 |
| MYRISTYL ALCOHOL | 0,0360 |
| ISOBUTYLPARABEN | 0,0204 |
| MYRETH-4 | 0,0180 |
| ASCORBYL PALMITATE | 0,0175 |
| SOLUBLE COLLAGEN | 0,0171 |
| ISOQUERCITRIN | 0,0150 |
| PROPYLPARABEN | 0,0110 |
| PROPYLENE GLYCOL | 0,0050 |
| CHITOSAN | 0,0046 |
| TOCOPHEROL | 0,0035 |
| SODIUM CHLORIDE | 0,0027 |
| SODIUM CHONDROITIN SULFATE | 0,0019 |
| GLUCOSE | 0,0019 |
| LYSINE HYDROCHLORIDE | 0,0006 |
| THREONINE | 0,0004 |
| ARGININE | 0,0004 |
| SERINE | 0,0002 |
| POTASSIUM CHLORIDE | 0,0002 |
| TRYPTOPHAN | 0,0001 |
| MAGNESIUM SULFATE | 0,0001 |
| GLYCINE | 0,0001 |
| CALCIUM CHLORIDE | 0,0001 |
| FOLIC ACID | 0,0001 |
| CALCIUM PANTOTHENATE | 0,0001 |

Hierin sind die Bestandteile HISTIDINE, GLUCOSE und CALCIUM CHLORIDE die Bestandteile der Wasserphase, welche als disperse Phase dient.

### Beispiel 2

| Formula (INCI/CTFA adopted names) | % w/w |
|---|---|
| WATER (AQUA) | 71,1509 |
| GLYCERIN | 4,3000 |
| METHYLGLUCOSE SESQUISTEARATE | 3,2200 |
| TOCOPHERYL ACETATE | 2,0050 |
| PPG-15 STEARYL ETHER | 2,0000 |
| ETHYLHEXYL METHOXYCINNAMATE | 2,0000 |
| PANTHENOL | 1,8750 |
| TRIISOSTEARIN | 1,7000 |
| SORBITAN STEARATE | 1,3800 |
| ETHYLHEXYL PALMITATE | 1,3000 |
| ISOPROPYL STEARATE | 1,2000 |
| CAPRYLIC/CAPRIC TRIGLYCERIDE | 1,2000 |
| MACADAMIA TERNIFOLIA SEED OIL | 1,0000 |
| DIMETHICONE | 0,8000 |
| PHENOXYETHANOL | 0,7556 |
| MYRISTYL MYRISTATE | 0,6000 |
| OCTYLDODECANOL | 0,5470 |
| CYCLOMETHICONE | 0,5000 |
| GLYCERYL POLYMETHACRYLATE | 0,4300 |
| IRVINGIA GABONENSIS KERNEL | |
| BUTTER | 0,3100 |
| IMIDAZOLIDINYL UREA | 0,3000 |
| XANTHAN GUM | 0,1700 |
| TITANIUM DIOXIDE | 0,1550 |
| METHYLPARABEN | 0,1571 |
| HYDROGENATED COCO-GLYCERIDES | 0,1350 |
| DISODIUM EDTA | 0,1000 |
| BHT | 0,1000 |
| ACRYLATES/C10-30 ALKYL ACRYLATE CROSSPOLYMER | 0,1000 |
| RETINYL PALMITATE | 0,0990 |
| TROMETHAMINE | 0,0700 |
| CETEARYL ALCOHOL | 0,0500 |
| BUTYLPARABEN | 0,0447 |
| ETHYLPARABEN | 0,0402 |
| LECITHIN | 0,0350 |
| SODIUM PCA | 0,0250 |
| BUTYLENE GLYCOL | 0,0209 |
| ISOBUTYLPARABEN | 0,0204 |
| ALUMINA | 0,0200 |
| SOLUBLE COLLAGEN | 0,0171 |
| ASCORBYL PALMITATE | 0,0125 |
| PROPYLPARABEN | 0,0111 |
| PROPYLENE GLYCOL | 0,0100 |
| SILICA | 0,0088 |
| SODIUM POLYACRYLATE | 0,0075 |
| CHITOSAN | 0,0046 |
| TOCOPHEROL | 0,0035 |

| Formula (INCl/CTFA adopted names) | % w/w |
|---|---|
| SODIUM CHLORIDE | 0,0027 |
| SODIUM CHONDROITIN SULFATE | 0,0019 |
| GLUCOSE | 0,0019 |
| LYSINE HYDROCHLORIDE | 0,0006 |
| THREONINE | 0,0004 |
| ARGININE | 0,0004 |
| SERINE | 0,0002 |
| POTASSIUM CHLORIDE | 0,0002 |
| HISTIDINE | 0,0002 |
| TRYPTOPHAN | 0,0001 |
| MAGNESIUM SULFATE | 0,0001 |
| GLYCINE | 0,0001 |
| CALCIUM CHLORIDE | 0,0001 |
| FOLIC ACID | 0,0001 |
| CALCIUM PANTOTHENATE | 0,0001 |

### Beispiel 3

| Formula (INCI/CTFA adopted names) | % w/w |
|---|---|
| WATER (AQUA) | 50,5510 |
| OCTOCRYLENE | 10,0000 |
| GLYCERIN | 7,5000 |
| CETEARYL ALCOHOL | 3,1000 |
| C12-15 ALKYL BENZOATE | 3,0000 |
| TITANIUM DIOXIDE | 2,4000 |
| TOCOPHERYL ACETATE | 2,0000 |
| DIETHYLHEXYL BUTAMIDO TRIAZONE | 2,0000 |
| CETEARETH-20 | 2,0000 |
| BIS-ETHYLHEXYLOXYPHENOL METHOXYPHENOL TRIAZINE | 2,0000 |
| HYDROGENATED COCO-GLYCERIDES | 1,0000 |
| GLYCERYL STEARATE | 0,7000 |
| PROPYLENE GLYCOL | 0,6250 |
| PEG-40 CASTOR OIL | 0,6000 |
| PHENOXYETHANOL | 0,5180 |
| FUMARIA OFFICINALIS (FUMITORY) EXTRACT | 0,5000 |
| CITRUS MEDICA LIMONUM (LEMON) EXTRACT | 0,5000 |
| MICA | 0,3240 |
| SODIUM CETEARYL SULFATE | 0,3000 |
| FRAGRANCE (PARFUM) | 0,3000 |
| DMDM HYDANTOIN | 0,2850 |
| XANTHAN GUM | 0,2000 |
| TRISODIUM EDTA | 0,1990 |
| FUMARIC ACID | 0,1250 |
| METHYLPARABEN | 0,1050 |
| TRIMETHOXYCAPRYLYLSILANE | 0,1000 |
| ETHYLPARABEN | 0,0280 |
| BUTYLPARABEN | 0,0280 |
| IODOPROPYNYL BUTYLCARBAMATE | 0,0150 |
| ISOBUTYLPARABEN | 0,0140 |
| PROPYLPARABEN | 0,0070 |
| IRON OXIDES | 0,4200 |
| TITANIUM DIOXIDE | 0,2400 |
| IRON OXIDES | 0,1960 |
| ULTRAMARINES | 0,0800 |
| IRON OXIDES | 0,0400 |
| Collagen | 0,03 |
| Chitosan | 0,01 |
| Chondroitinsulfat | 0,005 |
| ARGININE | 0,0004 |
| SERINE | 0,0002 |
| POTASSIUM CHLORIDE | 0,0002 |
| HISTIDINE | 0,0002 |
| TRYPTOPHAN | 0,0001 |
| MAGNESIUM SULFATE | 0,0001 |
| GLYCINE | 0,0001 |
| CALCIUM CHLORIDE | 0,0001 |
| FOLIC ACID | 0,0001 |

### Beispiel 4 - O/W Emulsion

KERATINOZYTENMEDIUM MCDB153 40 Gew.% KOLLAGEN/CHITOSAN/CHONDROITINSULFAT Matrix 6 Gew.% sowie in beliebiger Abmischung
WATER (AQUA)
GLYCERIN
HYDROGENATED COCO-GLYCERIDES
SQUALANE
GLYCERYL STEARATE CITRATE
CAPRYLIC/CAPRIC TRIGLYCERIDE
ETHYLHEXYL COCOATE
MYRISTYL ALCOHOL
BUTYROSPERMUM PARKII (SHEA BUTTER)
BUTYLENE GLYCOL
CETYL ALCOHOL
TOCOPHERYLACETATE
PHENOXYETHANOL
SODIUM CHLORIDE
IMIDAZOLIDINYL UREA
CARBOMER
XANTHAN GUM
METHYLPARABEN
EDTA
SODIUM HYDROXIDE
BHT
ETHYLPARABEN
BUTYLPARABEN
ISOBUTYLPARABEN
PROPYLPARABEN

### Beispiel 5 W/O/W-Emulsion

| | Gew.% |
|---|---|
| PEG-100-Stearat | 2,00 % |
| Glycerylstearat | 4,00 % |
| Squalan | 1,50 % |
| Squalen | 1,50 % |
| Isopropylpalmitat | 5,40 % |
| MCDB 153/DME 1:1 | 0,360 % |
| Magnesiumsulfat | 0,240 % |
| Konservierungsmittel | 0,50 % |
| KOLLAGEN/CHONDROITINSULFAT/CHITOSAN | 5% |
| Wasser VES | ad 100,00 % |

Die Fettphase, welche den Emulgator enthält, wird auf 80° C erhitzt, die Wasserphase ebenfalls, ohne denjenigen Anteil, der das Medium enthält. Bei 80° C werden beide Phasen miteinander vereinigt, ca. 3 - 10 Minuten lang homogenisiert und dann auf 48°C oder Raumtemperatur abgekühlt. Anschließend wird mit einer Temperaturkonstanz von ± 1 °C der Wasseranteil mit Medium zugesetzt und vermischt.

### Beispiel 6

| | |
|---|---|
| PEG-40-Stearat | 1,00 % |
| Glycerylstearat | 2,00 % |
| Cetylalkohol | 3,00 % |
| Mineralöl DAB 9 | 2,00 % |
| Safloröl | 2,00 % |
| Isopropylpalmitat | 4,50 % |
| Glycerin | 3,00 % |
| Magnesiumsulfat | 1,20 % |
| Konserv.-Mittel | 0,50 % |
| Kollagen/Chitosan/Glykosylaminoglykan-MATRIX | 2,00 % |
| Wasser VES | ad 100,00 % |
| davon DMEM/HAM's F-12 (1:1) | 2,5 % |

### Beispiel 7

| | |
|---|---|
| PEG-80-Stearat | 2,00 % |
| Cetylalkohol | 3,00 % |
| Mineralöl DAB 9 | 1,50 % |
| Nachtkerzenöl | 2,50 % |
| Isopropylpalmitat | 5,40 % |
| Propylenglycol | 3,00 % |
| Kaliumchlorid | 0,60 % |
| Konservierungsmittel | 0,50 % |
| Kollagen/Chitosan/Glykosylaminoglykan-MATRIX | 1,50 % |
| Wasser VES | ad 100,00 % |
| davon DMEM/HAM's F-12 (1:1) | 5% |

### Beispiel 8

| | |
|---|---|
| Steareth-100 | 2,00 % |
| Myristylalkohol | 1,00 % |
| Mineralöl DAB 9 | 3,00 % |
| Rizinusöl | 3,00 % |
| Cyclomethicone | 2,00 % |
| Propylenglycol | 3,00 % |
| Glycerin | 5,00 % |
| Kaliumchlorid | 3,00 % |
| Kollagen/Chitosan/Glykosylaminoglykan-MATRIX | 4,50 % |
| Konservierungsmittel | 0,50 % |
| Wasser VES | ad 100,00 % |
| davon MCDB 153 | 0,5 % |

### Beispiel 9

| | |
|---|---|
| Steareth-20 | 2,00 % |
| Cetearyl | 3,00 % |
| Vaseline | 0,50 % |
| Weizenkeimöl | 1,50 % |
| Dimethicone | 5,00 % |
| Glycerin | 5,00 % |
| Natriumchlorid | 3,00 % |
| Konservierungsmittel | 0,50 % |
| PUR | 1,50 % |
| KollagenlChitosan/Glykosylaminoglykan-MATRIX | 5,65 % |
| Wasser VES | ad 100,00 % |
| davon DMEM/HAM's F-12 (1:1) | 15 % |

### Beispiel 10

| | |
|---|---|
| Dimethicon Copolyol | 2,00 % |
| Cetearylalkohol | 3,00 % |
| Vaseline | 0,50 % |
| Weizenkeimöl | 1,50 % |
| Dimethicone | 5,00 % |
| Glycerin | 5,00 % |
| Natriumchlorid | 3,00 % |
| Konservierungsmittel | 0,50 % |
| Kollagen/Chitosan/Glykosylaminoglykan-MATRIX | 6,05 % |
| Wasser VES | ad 100,00 % |
| davon MCDB 153 | 1,5 % |

### Beispiel 11

| | |
|---|---|
| PEG-20-Behenat | 2,00 % |
| Stearylalkohol | 3,00 % |
| Vaseline | 1,00 % |
| Traubenkernöl | 3,00 % |
| Dimethicone | 3,00 % |
| Sorbit | 5,00 % |
| Zinksulfat | 3,00 % |
| Konservierungsmittel | 0,50 % |
| Kollagen/Chitosan/Glykosylaminoglykan-MATRIX | 10,5 % |
| Wasser VES | ad 100,00 % |
| davon MCDB 153 | 5 % |

### Beispiel 12

| | |
|---|---|
| Decaglyn 1-IS | 2,00 % |
| Stearylalkohol | 3,00 % |
| Vaseline | 1,00 % |
| Traubenkernöl | 3,00 % |
| Dimethicone | 3,00 % |
| Sorbit | 5,00 % |
| Zinksulfat | 3,00 % |
| Konservierungsmittel | 0,50 % |
| Kollagen/Chitosan/Glykosylaminoglykan-MATRIX | 25,00 % |
| Wasser VES | ad 100,00 % |
| davon MCDB 153 | 40% |

### Beispiel 13

| | |
|---|---|
| PEG-20-Myristat | 2,00 % |
| Stearylalkohol | 3,00 % |
| Vaseline | 2,00 % |
| Rizinusöl | 5,00 % |
| Dimethicone | 5,00 % |
| Sorbit | 5,00 % |
| Zinksulfat | 3,00 % |
| Konservierungsmittel | 0,50 % |
| Kollagen/Chitosan/Glykosylaminoglykan-MATRIX | 3,5 % |
| Wasser VES | ad 100,00 % |
| davon MCDB 153/RPMI 1640/Serumersatzstoffe | 0,1 % |

### Beispiel 14

| | |
|---|---|
| Sucroselaurat | 2,00 % |
| Stearylalkohol | 3,00 % |
| Vaseline | 2,00 % |
| Rizinusöl | 5,00 % |
| Dimethicone | 5,00 % |
| Sorbit | 5,00 % |
| Zinksulfat | 3,00 % |
| Konservierungsmittel | 0,50 % |
| Kollagen/Chitosan/Glykosylaminoglykan-MATRIX | 8,50 % |
| Wasser VES | ad 100,00 % |
| davon MCDB 153 | 12 % |

### Beispiel 15

| | |
|---|---|
| PEG-80-Behenat | 2,00 % |
| Glycerylbehenat | 4,00 % |
| Squalan | 3,00 % |
| Rizinusöl | 5,40 % |
| Glycerin | 6,00 % |
| Magnesiumsulfat | 2,60 % |
| Konservierungsmittel | 0,50 % |
| Kollagen/Chitosan/Glykosylaminoglykan-MATRIX | 0,0655 |
| Wasser VES | ad 100,00 % |
| davon MCDB 153/DME | 8,5 % |

### Beispiel 16 (O/W-Emulsion):

| | Gew.-% |
|---|---|
| Glycerylstearatcitrat | 3,00 |
| Stearylalkohol | 1,00 |
| Octyldodecanol | 1,00 |
| Caprylic/Capric Triglyceride | 1,00 |
| Dicaprylylether | 1,00 |
| Carbomer | 0,15 |
| Glycerin | 3,00 |
| Parfüm, Konservierungsmittel, NaOH Farbstoffe, Antioxidantien etc. | q.s. |
| Kollagen/Chitosan/Glykosylaminoglykan-MATRIX | 4,0 % |
| Wasser | ad 100,00 |
| davon DMEM/HAM's F-12 (1:1) | 2,5 % |
| pH-Wert eingestellt auf | 5,5 |

### Beispiel 17 (O/W-Emulsion):

| | Gew.-% |
|---|---|
| Glycerylstearatcitrat | 3,00 |
| Stearylalkohol | 1,00 |
| Octyldodecanol | 0,25 |
| Caprylic/Capric Triglyceride | 0,25 |
| Dicaprylylether | 0,25 |
| Carbomer | 0,15 |
| Glycerin | 3,00 |
| Parfüm, Konservierungsmittel, NaOH Farbstoffe, Antioxidantien etc. | q.s. |
| Kollagen/Chitosan/Glykosylaminoglykan-MATRIX | 5,0% |
| Wasser | ad 100,00 |
| davon MCDB 153 | 0,5 % |
| pH-Wert eingestellt auf | 5,5 |

### Beispiel 18 (ONV-Emulsion):

| | Gew.-% |
|---|---|
| Glycerylstearatcitrat | 3,00 |
| Behenylalkohol | 1,00 |
| Dimethicon | 1,50 |
| Cycolmethicon | 1,50 |
| Carbomer | 0,15 |
| Glycerin | 6,00 |
| Parfüm, Konservierungsmittel, NaOH Farbstoffe, Antioxidantien etc. | q.s. |
| Kollagen/Chitosan/Glykosylaminoglykan-MATRIX | 7,5 % |
| Wasser | ad 100,00 |
| davon DMEM/HAM's F-12 (1:1) | 5% |
| pH-Wert eingestellt auf | 5,5 |

### Beispiel 19 (O/W-Emulsion):

| | Gew.-% |
|---|---|
| Glycerylstearatcitrat | 3,00 |
| Stearylalkohol | 1,00 |
| Octyldodecanol | 0,25 |
| Caprylic/Capric Triglyceride | 0,25 |
| Dicaprylylether | 0,25 |
| Dimethicon | 0,50 |
| Carbomer | 0,15 |
| Glycerin | 3,00 |
| Aluminium Starch Octenyl Succinate | 0,50 |
| Talkum | 0,50 |
| Bentonite | 0,10 |
| Kollagen/Chitosan/Glykosylaminoglykan-MATRIX | 0,40 |
| Parfüm, Konservierungsmittel, NaOH Farbstoffe, Antioxidantien etc. | q.s. |
| Wasser | ad 100,00 |
| davon MCDB 153 | 80 % |
| pH-Wert eingestellt auf | 5,5 |

### Beispiel 20 (O/W-Emulsion):

| | Gew.-% |
|---|---|
| Glycerylstearatcitrat | 3,00 |
| Cetylalkohol | 1,00 |
| Squalan | 1,00 |
| Jojoba Öl | 1,00 |
| Paraffinum liquidum | 1,00 |
| Carbomer | 0,10 |
| Glycerin | 3,00 |
| Serin | 0,50 |
| Tocopherolacetat | 1,00 |
| Carbomer | 0,10 |
| Xanthangummi | 0,10 |
| Kollagen/Chitosan/Glykosylaminoglykan-MATRIX Parfüm, Konservierungsmittel, NaOH | 1,0% |
| Farbstoffe, Antioxidantien etc. | q.s. |
| Wasser | ad 100,00 |
| davon MCDB 153 | 75,5 % |
| pH-Wert eingestellt auf | 6,0 |

### Beispiel 21 (O/W-Emulsion):

| | Gew.-% |
|---|---|
| Glycerylstearatcitrat | 3,00 |
| Cetylalkohol | 0,50 |
| Octyldodecanol | 0,40 |
| Caprylic/Capric Triglyceride | 0,40 |
| Dicaprylylether | 0,40 |
| Carbomer | 0,10 |
| Glycerin | 3,00 |
| Serin | 0,50 % |
| Kollagen/Chitosan/Glykosylaminoglykan-MATRIX Parfüm, Konservierungsmittel, NaOH | 1,2% |
| Farbstoffe, Antioxidantien etc. | q.s. |
| Wasser | ad 100,00 |
| davon MCDB 153 | 45,5 % |
| pH-Wert eingestellt auf | 5,5 |

### Beispiel 22 (Emulsions-Make-up):

| | Gew.-% |
|---|---|
| Glycerylstearatcitrat | 3,00 |
| Stearylalkohol | 1,00 |
| Dimethicon | 0,50 |
| Glycerin | 1,50 |
| 1,3 Butylenglycol | 1,50 |
| Magnesiumsilikat | 1,00 |
| Glimmer | 1,00 |
| Eisenoxide | 1,00 |
| Titandioxid | 2,50 |
| Talkum | 5,00 |
| Carbomer | 0,15 |
| Kollagen/Chitosan/Glykosylaminoglykan-MATRIX | 0,50 |
| Parfüm, Konservierungsmittel, NaOH, Farbstoffe, Antioxidantien etc. | q.s. |
| Wasser | ad 100,00 |
| davon MCDB 153/Ham's F12/RPMI 1640 | 0,5 % |
| pH-Wert eingestellt auf | 5,5 |

### Beispiel 23 (O/W-Emutsion):

| | Gew.-% |
|---|---|
| Glycerylstearatcitrat | 3,00 |
| Stearylalkohol | 1,00 |
| Octyldodecanol | 0,25 |
| Caprylic/Capric Triglyceride | 0,25 |
| Dicaprylylether | 0,25 |
| Octylmethoxycinnamat | 4,00 |
| Benzophenone-3 | 3,00 |
| Octylsalicylat | 3,00 |
| Carbomer | 0,15 |
| Glycerin | 3,00 |
| Kollagen/Chitosan/Glykosylaminoglykan-MATRIX | 1,75 |
| Parfüm, Konservierungsmittel, NaOH Farbstoffe, Antioxidantien etc. | q.s. |
| Wasser | ad 100,00 |
| davon MCDB 153 | 40 % |
| pH-Wert eingestellt auf | 5,5 |

### Beispiel 23 (O/W-Emulsion):

| | Gew.-% |
|---|---|
| Glycerylstearatcitrat | 3,00 |
| Stearylalkohol | 1,00 |
| Octyldodecanol | 0,50 |
| Caprylic/Capric Triglyceride | 0,50 |
| Dicaprylylether | 0,50 |
| Distärkephosphat | 1,00 |
| Ethanol | 10,00 |
| Carbomer | 0,15 |
| Glycerin | 3,00 |
| Kollagen/Chitosan/Glykosylaminoglykan-MATRIX | 0,0625 % |
| Parfüm, Konservierungsmittel, NaOH Farbstoffe, Antioxidantien etc. | q.s. |
| Wasser | ad 100,00 |
| davon MCDB 153 | 35 % |
| pH-Wert eingestellt auf | 5,5 |

### Beispiel 24 (Emulgatorgel):

| | Gew.-% |
|---|---|
| Glycerylstearatcitrat | 3,00 |
| Stearylalkohol | 1,00 |
| Ethanol | 2,00 |
| Aluminium Starch Octenyl Succinate | 0,25 |
| Talkum | 0,25 |
| Tapiokastärke | 0,25 |
| Carbomer | 0,15 |
| Glycerin | 3,00 |
| Kollagen/Chitosan/Glykosylaminoglykan-MATRIX | 0,10 |
| Parfüm, Konservierungsmittel, NaOH Farbstoffe, Antioxidantien etc. | q.s. |
| Wasser | ad 100,00 |
| davon DMEM/HAM's F-12 (1:1) | 3,5 % |
| pH-Wert eingestellt auf | 5,5 |

## Patentansprüche

1. Kosmetische Zubereitung umfassend Kollagen, Chitosan mit einem Acetylierungsgrad bis zu 50% und Glykosylaminoglykan auf Basis von wässrigen Gelen, Emulsionen vom O/W-, W/O/W- oder W/O-Typ, Mikroemulsionen oder kosmetischen Stiftpräparaten, wobei Kollagen und Chitosan im Verhältnis 60 bis 90% zu 40 bis 10% enthalten sind.

2. Zubereitung nach Anspruch 1 **dadurch gekennzeichnet, dass** das eingesetzte Kollagen und/oder Chitosan durch maritime- und/oder synthetische Rohstoffquellen gewonnen wurde.

3. Zubereitung nach einem der Ansprüche 1 oder 2 **dadurch gekennzeichnet, dass** das Kollagen aus maritimen Kollagenen, gewählt aus der Gruppe des Typ 3, Typ 1, Typ 4 und/oder Typ 5 oder Abmischungen derselben, besteht.

4. Zubereitung nach einem der Ansprüche 1 bis 3 **dadurch gekennzeichnet, dass** das eingesetzte Chitosan aus Krustentieren und/oder Insekten gewonnen wurde.

5. Zubereitung nach einem der vorstehenden Ansprüche **dadurch gekennzeichnet, dass** das Chitosan ein Molgewicht von 80.000 g/mol bis 1,5.000.000 g/mol aufweist.

6. Zubereitung nach einem der vorstehenden Ansprüche **dadurch gekennzeichnet, dass** das Glykosylaminoglykan ausgewählt wird aus Chondroitin-4- und/oder Chondroitin-6-Sulfat.

7. Zubereitung nach einem der vorstehenden Ansprüche enthaltend die Kollagen, Chitosan und Glykosylaminoglykan-Matrix zu 0,00001 Gew.% bis 99 Gew.%, vorzugsweise 0,0005 Gew.% bis 50 Gew.%, besonders bevorzugt 0,0015 bis 30 Gew.%, bezogen auf die Gesamtmasse der Zubereitung.

8. Zubereitung nach einem der vorstehenden Ansprüche enthaltend zusätzlich mindestens eine Verbindung, bevorzugt alle, gewählt aus der Gruppe L-Cystin, L-Glutamin, Hypoxanthin, L-Glutaminsäure, Thymidin, Glycin, Liponsäure, L-Histidin-HCl, Linolsäure, L-Isoleucin, Putrescin-2HCl, NaCl, Cholinchlorid, KCl, Putrescin, Natriumacetat, Vitamin B12, Na2HPO4. Biotin, MgCl2, Calciumpantothenat, CaCl2, Nicotinamid, Glucose, Pyridoxin-HCl, Natriumpyruvat, Thiamin-HCl, NaHC03, Adenin, Phenolrot, myo-Inositol, HEPES, Liponsäure, Thymidin, L-Alanin, Folsäure, L-Arginin-HCl, Riboflavin, L-Asparagin, L-Asparaginsäure, CuSO4, L-Cystein-HCl, FeSO4, L-Glutamin, MnSO4, L-Glutaminsäure, (NH4)6Mo7O24, Glycin, NiCl2, L-Histidin-HCl, H2SeO3, L-Isoleucin, Na2SiO3, L-Leucin, SnCl2, L-Lysin- HCl, NH4VO3, L-Methionin, ZnSO₄, L-Phenylalanin, L-Prolin, L-Serin, L-Threonin, L-Tryptophan, L-Tyrosin, L-Valin, FeSO4, Insulin human, ZnSO4, CoCl2, CuSO4, Na2SeO3, AlCl3, CrK(SO4)2, NiCl3, MnCl2, EDTA.Na2, Polysorbat 80, L-Leucin, Na2HPO4, L-Methionin, NaH2PO4, L-Phenylalanin, MgSO4, MgCl2, L-Serin, CaCl2, L-Threonin, Fe(NO3)3, L-Tryptophan, FeSO4, L-Tyrosin, NaHCO3, α-Biotin, Na-Pyruvat, Folsäure, D-Ca-Pantothenat, L-Alanin, L-Arginin-HCl und/oder Pyridoxal-HCl.

9. Zubereitung nach einem der vorstehenden Ansprüche **dadurch gekennzeichnet, dass** aus den Bestandteilen Kollagen, Chitosan und Glykosylaminoglykan eine Nano- oder Mikroschwammmatrix in Zellkulturmedien rekonstituiert ist.

10. Zubereitung nach einem der Ansprüche enthaltend Kollagen und Chitosan im Verhältnis 75 - 85% zu 25 - 15%.

11. Zubereitung nach Anspruch 9 **dadurch gekennzeichnet, dass** das Zellkulturmedium gewählt wird aus physiologischer Kochsalzlösung, Nährmedium oder Vollmedium zur Aufzucht von gesunden, primären Körperzellen.

12. Zubereitung nach Anspruch 11 **dadurch gekennzeichnet, dass** die Medien aus der Gruppe der Kulturmedien für primäre Fibroblasten und Keratinozyten stammen.

13. Zubereitung nach Anspruch 9, 10 oder 11 **dadurch gekennzeichnet, dass** die Vollmedien mit Serumersatzstoffen supplementiert sind.

14. Zubereitung nach einem der vorstehenden Ansprüche enthaltend zusätzlich ein oder mehrere Hautzellkulturmedien, in denen das Medien bevorzugt gewählt wird aus DMEM/HAM's F-12 (1:1) und/oder MCDB 153.

15. Zubereitung nach einem der vorstehenden Ansprüche zusätzlich enthaltend einen Citratpuffer.

16. Zubereitung nach einem der vorstehenden Ansprüche enthaltend zusätzlich Q10, AGR, Zn-Orotrat, Carnitin, Kreatin und/oder Taurin.

## Claims

1. Cosmetic preparation including collagen, chitosan with a degree of acetylation of up to 50% and glycosylaminoglycan based on aqueous gels, emulsions of the o/w, w/o/w or w/o type, microemulsions or cosmetic stick products, wherein it comprises collagen and chitosan in the ratio 60 to 90% to 40 to 10%.

2. Preparation according to Claim 1, **characterized in that** the collagen and/or chitosan employed has been obtained through marine and/or synthetic raw materials sources.

3. Preparation according to either of Claims 1 or 2, **characterized in that** the collagen consists of marine collagens selected from the group of type 3, type 1, type 4 and/or type 5 or blends thereof.

4. Preparation according to any of. Claims 1 to 3, **characterized in that** the chitosan employed has been obtained from crustaceans and/or insects.

5. Preparation according to any of the preceding claims, **characterized in that** the chitosan has a molecular weight of from 80 000 g/mol to 1.5 000 000 g/mol.

6. Preparation according to any of the preceding claims, **characterized in that** the glycosylaminoglycan is selected from chondroitin 4-sulfate and/or chondroitin 6-sulfate.

7. Preparation according to any of the preceding claims comprising the collagen, chitosan and glycosylaminoglycan matrix as from 0.00001% by weight to 99% by weight, preferably 0.0005% by weight to 50% by weight, particularly preferably 0.0015 to 30% by weight of the total mass of the preparation.

8. Preparation according to any of the preceding claims, comprising additionally at least one compound, preferably all selected from the group of L-cysteine, L-glutamine, hypoxanthine, L-glutamic acid, thymidine, glycine, lipoic acid, L-histidine HCl, linoleic acid, L-isoleucine, putrescine 2HCl, NaCl, choline chloride, KCl, putrescine, sodium acetate, vitamin B₁₂, Na₂HPO₄, biotin, MgCl₂, calcium pantothenate, CaCl₂, nicotinamide, glucose, pyridoxine HCl, sodium pyruvate, thiamine HCl, NaHC03, adenine, phenol red, myo-inositol, HEPES, lipoic acid, thymidine, L-alanine, folic acid, L-arginine HCl, riboflavin, L-asparagine, L-aspartic acid, CuSO₄, L-cysteine HCl, FeSO4, L-glutamine, MnSO4, L-glutamic acid, (NH₄)₆Mo₇O₂₄, glycine, NiCl₂, L-histidine HCl; H₂SeO₃, L-isoleucine, Na₂SiO₃, L-leucine, SnCl₂, L-lysine HCl, NH₄VO₃, L-methionine, ZnSO₄, L-phenylalanine, L-proline, L-serine, L-threonine, L-tryptophan, L-tyrosine, L-valine, FeSO4, insulin human, ZnSO₄, CoCl₂, CuSO₄, , Na₂SeO₃, AlCl₃, CrK(SO₄)₂, NiCl₃, MnCl₂, EDTA.Na₂, polysorbate 80, L-leucine, Na₂HPO₄, L-methionine, NaH₂PO₄, L-phenylalanine, MgSO₄, MgCl₂, L-serine, CaCl₂, L-threonine, Fe(NO₃)₃, L-tryptophan, FeSO₄, L-tyrosine, NaHCO₃, α-biotin, Na pyruvate, folic acid, D-Ca pantothenate, L-alanine, L-arginine HCl and/or pyridoxal HCl.

9. Preparation according to any of the preceding claims, **characterized in that** a nano sponge or micro sponge matrix is reconstituted from the collagen, chitosan and glycosylaminoglycan constituents in cell culture media.

10. Preparation according to any of the claims, comprising collagen and chitosan in the ratio 75-85% to 25-15%.

11. Preparation according to Claim 9, **characterized in that** the cell culture medium is selected from physiological saline solution, nutrient medium or complete medium for culturing healthy, primary body cells.

12. Preparation according to Claim 11, **characterized in that** the media are derived from the group of culture media for primary fibroblasts and keratinocytes.

13. Preparation according to Claim 9, 10 or 11, **characterized in that** the complete media are supplemented with serum substitutes.

14. Preparation according to any of the preceding claims, comprising additionally one or more skin cell culture media in which the media is preferably chosen from DMEM/HAM's F-12 (1:1) and/or MCDB 153.

15. Preparation according to any of the preceding claims additionally comprising a citrate buffer.

16. Preparation according to any of the preceding claims comprising additionally Q10, AGR, Zn orotrate, carnitine, creatine and/or taurine.

## Revendications

1. Composition cosmétique comprenant du collagène, du chitosane ayant un degré d'acétylation de jusqu'à 50 % et du glycosylaminoglycane, à base de gels aqueux, d'émulsions du type H/E, E/H/E ou E/H, de micro-émulsions ou de préparations pour crayons cosmétiques, contenant du collagène et du chitosane en un rapport de 60 à 90% : 40 à 10%.

2. Composition selon la revendication 1, **caractérisée en ce que** le chitosane et/ou le collagène utilisé(s) a/ont été obtenu(s) à partir de sources de matières premières marines et/ou synthétiques.

3. Composition selon l'une quelconque des revendications 1 et 2, **caractérisée en ce que** le collagène consiste en des collagènes marins, choisis dans le groupe constitué par le type 3, le type 1, le type 4 et/ou le type 5 ou des mélanges de ceux-ci.

4. Composition selon l'une quelconque des revendications 1 à 3, **caractérisée en ce que** le chitosane utilisé a été obtenu à partir de crustacés et/ou d'insectes.

5. Composition selon l'une quelconque des revendications précédentes, **caractérisée en ce que** le chitosane présente une masse moléculaire de 80 000 g/mole à 1, 5.000.000 g/mole.

6. Composition selon l'une quelconque des revendications précédentes, **caractérisée en ce que** le glycosylaminoglycane est choisi parmi le 4-sulfate de chondroïtine et/ou le 6-sulfate de chondroïtine.

7. Composition selon l'une quelconque des revendications précédentes, contenant la matrice de collagène, chitosane et glycosylaminoglycane à raison de 0,00001 % en poids à 99 % en poids, de préférence de 0,0005 % en poids à 50 % en poids, de façon particulièrement préférée de 0,0015 à 30% en poids, par rapport à la masse totale de la composition.

8. Composition selon l'une quelconque des revendications précédentes, contenant en outre au moins un composé, de préférence tous les composés, choisi(s) dans le groupe constitué par la L-cystine, la L-glutamine, l'hypoxanthine, l'acide L-glutamique, la thymidine, la glycine, l'acide lipoïque, la L-histidine-HCl, l'acide linoléique, la L-isoleucine, la putrescine-2HCl, NaCl, le chlorure de choline, KCl, la putrescine, l'acétate de sodium, la vitamine B₁₂, Na₂HPO₄, la biotine, MgCl₂, le pantothénate de calcium, CaCl₂, le nicotinamide, le glucose, la pyridoxine-HCl, le pyruvate de sodium, la thiamine-HCl, NaHCO₃, l'adénine, le rouge de phénol, le myo-inositol, HEPES, l'acide lipoïque, la thymidine, la L-alanine, l'acide folique, la L-arginine-HCl, la riboflavine, la L-asparagine, l'acide L-aspartique, CuSO₄, la L-cystéine-HCl, FeSO4, la L-glutamine, MnSO4, l'acide L-glutamique, (NH₄)₆Mo₇O₂₄, la glycine, NiCl₂, la L-histidine-HCl, H₂SeO₃, la L-isoleucine, Na₂SiO₃, la L-leucine, SnCl₂, la L-lysine-HCl, NH₄VO₃, la L-méthionine, ZnSO₄, la L-phénylalanine, la L-proline, la L-sérine, la L-thréonine, le L-tryptophane, la L-tyrosine, la L-valine, FeSO4, l'insuline humaine, ZnSO₄, CoCl₂, CuSO₄, Na₂SeO₃, AlCl₃, CrK(SO₄)₂, NiCl₃, MnCl₂, EDTA.Na₂, le polysorbate 80, la L-leucine, Na₂HPO₄, la L-méthionine, NaH₂PO₄, la L-phénylalanine, MgSO₄, MgCl₂, la L-sérine, CaCl₂, la L-thréonine, Fe(NO₃)₃, le L-tryptophane, FeSO4, la L-tyrosine, NaHCO₃, l'α- biotine, le pyrivate de sodium, l'acide folique, le D-pantothénate de Ca, la L-alamine, la L-arginine-HCl et/ou le pyridoxal-HCl.

9. Composition selon l'une quelconque des revendications précédentes, **caractérisée en ce qu'**à partir des composants collagène, chitosane et glycosylaminoglycane on reconstitue une matrice nano-ou micro-spongieuse dans des milieux de culture de cellules.

10. Composition selon l'une quelconque des revendications, contenant du collagène et du chitosane en un rapport de 75-85% : 25-15%.

11. Composition selon la revendication 9, **caractérisée en ce que** le milieu de culture de cellules est choisi parmi une solution physiologique de chlorure de sodium, un milieu nutritif ou un milieu complet pour la culture de cellules corporelles primaires, saines.

12. Composition selon la revendication 11, **caractérisée en ce que** les milieux proviennent du groupe des milieux de culture pour kératinocytes et fibroblastes primaires.

13. Composition selon la revendication 9, 10 ou 11, **caractérisée en ce que** les milieux complets sont complémentés avec des substituts de sérum.

14. Composition selon l'une quelconque des revendications précédentes, contenant en plus un ou plusieurs milieux de culture de cellules de la peau, dans lesquels le milieu est choisi de préférence parmi DMEM/HAM F-12 (1:1) et/ou MCDB 153.

15. Composition selon l'une quelconque des revendications précédentes, contenant en plus un tampon citrate.

16. Composition selon l'une quelconque des revendications précédentes, contenant en plus Q10, AGR, de l'orotrate de Zn, de la carnitine, de la créatine et/ou de la taurine.
